# EUROPEAN PATENT SPECIFICATION

(11) **EP 3 487 388 B1**
(45) Date of publication and mention of the grant of the patent: **10.09.2025**
(21) Application number: 17830504.1
(22) Date of filing: 21.07.2017
(51) Int. Cl.: A61B 5/021, A61B 5/022, A61B 5/024, A61B 5/145, A61B 5/00

(54) **AN ELECTRONIC DEVICE FOR MEASURING PHYSIOLOGICAL INFORMATION AND A METHOD THEREOF**
ELEKTRONISCHE VORRICHTUNG ZUR MESSUNG PHYSIOLOGISCHER INFORMATIONEN UND VERFAHREN DAFÜR
DISPOSITIF ÉLECTRONIQUE POUR MESURER DES INFORMATIONS PHYSIOLOGIQUES ET PROCÉDÉ ASSOCIÉ

(30) Priority: 22.07.2016 US 201662365978 P
(43) Date of publication of application: 29.05.2019
(73) Proprietor: Amorv (IP) Company Limited, N.T. Hong Kong (HK)
(72) Inventor: CHEN, Hung Tat, Tai Koo Shing Hong Kong (CN); GU, Wenbo, Kowloon Hong Kong (CN); TO, Kwan Wai, Kowloon Hong Kong (CN); LAP, Wai Lydia Leung, Kowloon Hong Kong (CN)
(74) Representative: Potter Clarkson
(86) International application number: PCT/CN2017/093917
(87) International publication number: WO 2018/014870

(56) References cited:
- EP-A1- 1 360 930
- WO-A1-2016/096391
- CN-A- 1 383 371
- CN-A- 1 456 125
- CN-A- 1 522 672
- CN-A- 102 613 966
- CN-A- 105 725 998
- US-A1- 2016 089 042
- US-A1- 2016 100 787

## Description

### PRIORITYOF RELATED APPLICATION

This application is based upon and claims priority to United States Patent Application No. 62365978, filed before United States Patent and Trademark Office on July 23, 2016 and entitled "AN ELECTRONIC DEVICE FOR MEASURING PHYSIOLOGICAL INFORMATION AND A METHOD THEREOF'.

### TECHNICAL FIELD

This invention relates to an electronic device that measures physiological information of a living subject.

### BACKGROUND

Nowadays, technology integrated with health tools are becoming a popular trend within the healthcare industry and are being used on a more regular basis. Many of the electronic devices are providing a plethora of health data from the growing roster of available tools that can be used by consumers for both personal and clinical decisions. Generally, the electronic devices with health tools could measure heart rate (HR), heart rate variability(HRV), blood pressure, temperature, motion, and/or other biological information of the user via a noninvasive method.

In one application field, an electronic device is designed to measure health data of a user, e.g., heart rate and blood pressure, via the blood vessel of the wrist. A cuff-type wrist blood pressure meter occludes all blood vessels around the wrist to measure the blood pressure. Hence, it cannot be used to measure continual blood pressure. In order to measure continual blood pressure, some electronic devices measure photoplethysmography (PPG) signal and electrocardiogram (ECG) signals to calculate pulse transit time (PTT) and estimate blood pressure accordingly. However, frequent calibration is needed for PTT-based blood pressure estimation. Also, it is inconvenient to measure both PPG and ECG.
In view of demand for measuring health data, improvements that provide an accurate and compact electronic device for continual blood pressure measurement are desired.
US 2016/0100787 A1 discloses techniques for blood pressure measurement with psychological status validation. CN 1522672 A discloses a stereo-directional heat therapy system in treating tumours in the interior of the human body.

### SUMMARY

According to aspects of the invention, there is provided an electronic device as recited in Claim 1 and a method as recited in Claim 10. Optional features of the invention are recited in the dependent claims.

Other example embodiments are discussed herein.

### BRIEF DESCRIPTION OF THE DRAWINGS

Figure 1 illustrates a preferred location on a wrist of a user 100 for measuring the blood pressure according to one example embodiment.
Figure 2 illustrates a block diagram of an electronic device 200 for healthcare, according to one example embodiment.
Figure 3 illustrates a schematic drawing of an electronic device 300 for healthcare, according to one example embodiment.
Figure 4 illustrates a schematic drawing of an electronic device 400 for detecting the blood pressure at an internal side of a user's wrist, according to one example embodiment.
Figure 5 shows a top view of the sensor assembly 206 used in the electronic device 200, according to one example embodiment.
Figure 6A and 6B show an operating mechanism of the electronic device with the sensor assembly 206 on the user's wrist, according to one example embodiment.
Figure 7A shows waveforms of a reflected optical signal and pressure pulse signal received by the sensor assembly 206 when it is in touch with the user's wrist as illustrated in Figure 6A, according to one example embodiment.
Figure 7B shows waveforms of a reflected optical signal and pressure pulse signal received by the sensor assembly 206 when it is pressed against the wrist surface as illustrated in Figure 6B, according to one example embodiment.
Figure 8A illustrates an exemplary schematic structure of the electronic device 200 with a membrane unit, according to one example embodiment.
Figure 8B shows the new membrane section 873 of the membrane unit from a bottom view, according to one example embodiment.
Figure 8C shows the electronic device 200 with the membrane unit contacting the user's skin, according to one example embodiment. Figure9A shows a top view of a sensor assembly 906 with a coating layer, according to one example embodiment.
Figure 9B shows a cross section view (from AA' direction of Figure 9A) of the sensor assembly 906 with the coating layer, according to one example embodiment.
Figure 10A shows a movable frame being worn on a user's wrist via a wristband, according to one example embodiment.
Figure 10B shows a portable device that disposes on the user's wrist and coupled to the movable frame for measuring the health information of the user, according to one example embodiment.
Figure 11 illustrates a measurement relationship between the sensed signal, the scanning trace and the skin contour, according to one example embodiment.
Figure 12illustrates a flowchart of predicting the measuring position via a prediction algorithm, according to one example embodiment.
Figure 13 illustrates a method of applying an electronic device to a user, in according to one example embodiment.

### DETAILED DESCRIPTION

Reference will now be made in detail to the embodiments of the present invention. While the invention will be described in conjunction with these embodiments, it will be understood that they are not intended to limit the invention to these embodiments. The invention is defined by the appended claims.

Furthermore, in the following detailed description of the present invention, numerous specific details are set forth in order to provide a thorough understanding of the present invention. However, it will be recognized by one of ordinary skill in the art that the present invention may be practiced without these specific details. In other instances, well known methods, procedures, components, and circuits have not been described in detail as not to unnecessarily obscure aspects of the present invention. In the light of the foregoing background, it is an object of the present invention to provide an electronic device for monitoring health status of the user.

In one example embodiment, an electronic device for healthcare is, but not limited to, a wrist-worn device that measures the health data, e.g., heart rate, heart rate variability, blood pressure, blood oxygen saturation, and/or stress, of a user. In one embodiment, the electronic device is a wristband that is rigid or flexible to be worn on the wrist and can have various shapes and sizes without departing from the scope of example embodiments. Other example embodiments can be worn on an arm, neck, leg, ankle, or other part of the human body.

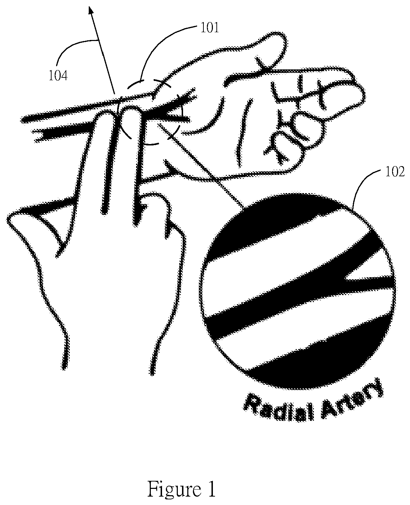

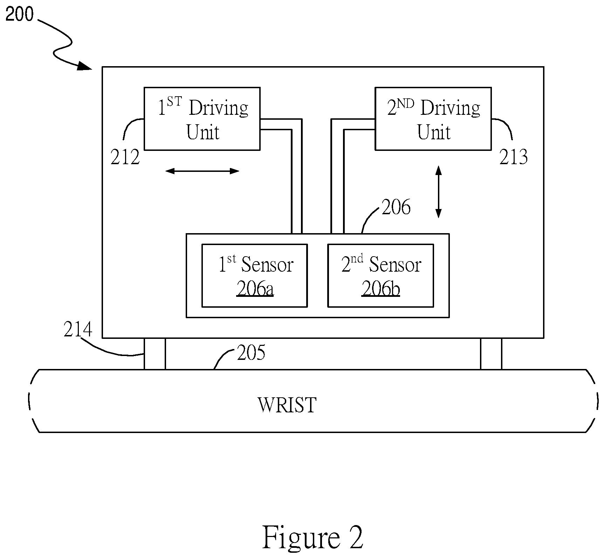

Figure 1 illustrates a location on a wrist 100 for measuring the blood pressure, according to one example embodiment. The electronic device includes a pressure sensor (shown in Fig. 2), which is to be located near an artery of the wrist, for measuring the blood pressure. More specifically, the pressure sensor is located near a radial artery superficially above the distal end of the radial bone, as exemplarily specified by a dot line circle 101. An enlarged image 102 of the radial artery within the circle 101 shows more details thereof, in which the pressure sensor is located within a neighboring region of the radial artery. When the radial artery is compressed against the radial bone by the pressure sensor, the pulse can be clearly sensed at the wrist where it is covered by thin skin and tissue. As such, a target neighboring region of the radial artery of the wrist, which is exemplarily specified as the circle area 101 to secure an accurate measurement, needs to be determined.

In some cases, a pressure sensor array is used to detect multiple pulse signals within a certain area, e.g., the circle area 101, and to select a largest one of which the location is near the radial artery of the wrist for accurately measuring the health data, e.g., heart rate and blood pressure, of the user. However, the pressure sensor array is bulky and expensive. In some cases, a motor is used to move a pressure sensor along the wrist surface in a predetermined direction, e.g., a direction 104 perpendicular to the artery direction as shown in Figure 1, to detect multiple pulse signals within the predetermined direction. Similarly, a largest detected signal is selected of which the corresponding location is determined for measuring the health data of the user. However, during the movement, since the pressure sensor is in touch with the skin surface, the motor with enough torque is needed to overcome the friction between the sensor and the skin surface. Step motors or DC motors are used for driving the pressure sensor to sweep along the wrist surface to obtain multiple pulse signals for identifying the optimal measuring position where an artery is predicted to lie thereunder. However, the step or DC motors are bulky which make the whole electronic device not compact and inconvenient for long-term use.

In order to overcome the above problems, a non-contact sensor, e.g., a wireless wave sensor, is adopted to move along the wrist surface to sense the physiological information of the user at multiple positions, that is, to scan the wrist skin, without, at least partially, contacting the skin surface (in a contactless way) and determine a measuring position based on the sensed physiological information, according to one example embodiment. In one example embodiment, the wireless wave sensor includes electromagnetic or mechanical wave sensor that is able to emit and detect electromagnetic or mechanical wave. In one example embodiment, the wireless wave sensor is an optical sensor. In one example embodiment, a non-contact scan region is around 15-20 mm. In one example embodiment, the measuring position is near to, at least within an acceptable neighboring range of, the target blood vessel under the wrist surface. In one example embodiment, the non-contact sensor emits a signal, e.g., an optical or ultrasound signal, toward the wrist surface and detects the signal reflected from the wrist during the movement. Based on the detected signal, the target measuring position is identified. A pressure sensor is then used to sense the physiological information based on the identified measuring position. In one embodiment, the accuracy of the measuring position identified by non-contact scanning is around 3 mm and then the pressure sensor needs to fine-tune the measuring position by sensing pressure pulse signals at multiple positions surround the identified measuring position and determine a more accurate position based on the sensed pressure pulse signals. In another embodiment, the accuracy of measuring position identified by non-contact scanning has been increased to within 1mm according to a prediction algorithm. In this case, the pressure sensor can directly sense the blood pressure at the measuring position identified by the non-contact scanning process. In one embodiment, since during the scanning process, the non-contact sensor is above the skin surface without, at least partially, contacting the skin surface, the torque needed to drive the non-contact sensor to move along the wrist surface is significantly reduced, as comparing with the torque needed to drive the pressure sensor to contact and move along the skin surface. Under such condition, a more compact driving unit, e.g., Voice Coil Motor (VCM), can be used to move the non-contact sensor along the wrist surface. The dimension of the VCM is much smaller than that of the other mechanical/electrical motor, e.g., step or DC motors. Furthermore, the VCM can control the tilting angle of the sensors mounted on the motor so that more accurate measurement can be achieved.

Figure 2 illustrates a block diagram of an electronic device 200 for healthcare, according to one example embodiment. As shown in Figure 2, when the electronic device 200 is worn on a user's wrist 216, it is mounted on the inner side 205 of the wrist 216 via the supporting unit 214. The electronic device 200 includes a sensor assembly 206 that is used for sensing the physiological information, e.g., pulse rate, blood pressure and blood oxygen saturation information, of the living object; a first driving unit 212, that drives the sensor assembly 206 to scan the wrist surface 205 within a predetermined region to detect the measuring position for measuring the health data of the user; and a second driving unit 213 that drives the sensor assembly 206 to move in a direction perpendicular or substantially perpendicular to the wrist surface 205 in order to keep a certain distance between the skin and the sensor assembly 206 when doing contactless scan and drives the sensor assembly 206 to contact and press against the wrist skin when sensing blood pressure pulsation and photoplethysmography waveform.

In one example embodiment, the sensor assembly 206 includes a first sensor 206a and a second sensor 206b, wherein the first sensor 206a is an optical sensor that detects the artery position of the wrist in a contactless way, and the second sensor 206b is a pressure sensor which is driven by a hold-down force to contact and press against the wrist surface 205 for fine-tuning measurement location and measuring the pressure against the wall of the artery. In one embodiment, the first sensor 206a emits light toward the wrist 216 and detects the light reflected from the wrist 216 while moving along a predetermined path, so as to determine the artery position based on the detected result. In one embodiment, the first sensor 206a and the second sensor 206b are integrated in the sensor assembly 206 and moved together in a first and second directions by the first driving unit 212 and the second driving unit 213. In an alternative example embodiment, the first sensor 206a and second sensor 206b are separated units. Under such condition, the first sensor 206a will be driven to contactlessly scan the wrist surface for determining the measuring position and the second sensor 206b will be driven to press against the skin to sense the blood pressure at the measuring position. However, for easy illustration and understanding, the embodiment that the first and second sensors 206a and 206b move together with the sensor assembly 206 will be used for later description and it is understood by people having ordinary skill in the art that other embodiments, e.g., the first and second sensors 206a and 206b, are separated and moved independently from each other, could be also applied to the following description with reasonable changes.

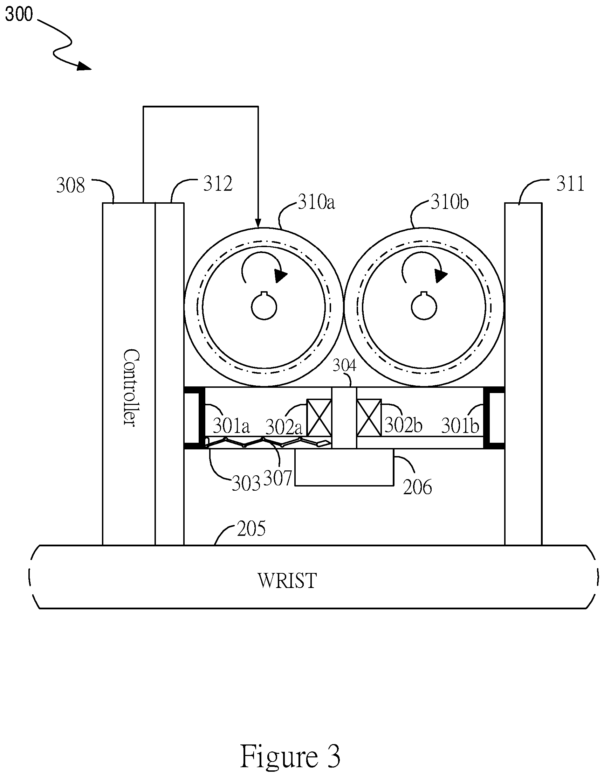

Figure 3 illustrates a schematic drawing of an electronic device 300 for healthcare, in accordance with one example embodiment. Figure 3 is described in combination with Figure 2. Elements with the same or similar reference numerals have the same or similar structure/function as thereof in previous figures. In the electronic device 300 of Figure 3, a guiding unit 314 couples to the sensor assembly 206 for guiding the sensor assembly 206 to scan the wrist surface 205 in a contactless way. In one example embodiment, the guiding unit 314 comprises at least one guiding rail 303 and at least one moving element 304 moving along the guiding rail 303. In one example embodiment, the moving element 304 is a rolling or sliding element. As understood by one skilled in ordinary of the art, the guiding unit 314 is not limited to the structure as illustrated in Figure 3 and could have alternative configurations. For example, the guiding rail 303 could guide the moving element 304 to move in a straight or curve orientation. The guiding rail 303 and the moving element 304 could be of any shape/structure as long as it satisfies the guiding function. Furthermore, the first driving unit 212 from Figure 2 (not specified in Figure 3) is an electromagnetic motor that includes at least one magnet 301a, and at least one coil 302a that interacts with the magnet 301a for generating an electromagnetic force. The electromagnetic motor couples to the guiding unit 314 for driving the guiding unit 314 based on the electromagnetic force to guide the sensor along a predetermined scanning path above the skin surface. In one example embodiment, the first driving unit 212 is a VCM motor.

In one example embodiment, the magnet 301a is fixed and the coil 302a is movable and mounts to the moving element 304. When a current flows through the coil 302a, an electromagnetic force is generated between the magnet 301a and the coil 302a to enable the coil 302a, together with the moving element 304, to move toward or away from the magnet 301a along the guiding rail 303. In an alternative example embodiment, the coil 302a is fixed and the magnet 301a is movable and attached to the moving element 304 in a similar structure. Furthermore, an elastic unit 307, with one end being coupled to the moving element 304, provides a restoring force to the moving element 304. In one example embodiment, the elastic unit 307 is a spring. In one example embodiment, the elastic unit 307 has one end that is fixed to the magnet 301a and the other end is coupled to the moving element 304. Under a combined effect of the electromagnetic force and the restoring force, the guiding unit 314 could guide the sensor assembly 206 to move toward and stay steadily at a target position when the current flows through the coils 302a. When no current flows through the coils 302a, the restoring force of the elastic unit 307 will bring the sensor assembly 206 back to its initial position.

In one example embodiment, a friction force between the guiding rail 303 and the moving element 304 is predefined to reduce the shift and improve the stability of the sensor assembly 206 while staying at the targeted position. In another example embodiment, two or more sets of the magnet and coil 301a/302a and 301b/302b dispose at two sides of the moving element 304 in order to provide pushing/pulling force at the two sides of the moving element 304 for enhancing movement control and improving stability. One of ordinary skill in the art may appreciate that details of the electronic device as discussed therein are merely examples. Other embodiments and details can be provided by the electronic device without departing from the scope of this invention. For example, the elastic unit 307 could be configured in any format at any place as long as it satisfies the requirement of providing a restoring force that corresponds to an electromagnetic force to bring the sensor assembly 206 back to its initial position when the electromagnetic motor is turned off. In one example embodiment the elastic unit 307 could be configured in any format at any place along the guiding rail 303.

In one example embodiment, the non-contact scanning process is performed by the first sensor 206a in a cross-artery direction and the distance between the first sensor 206a and the skin surface is controlled to be within 1-2 mm. In one embodiment, the first driving unit 212 will control the movement of the sensor assembly 206 to perform the scanning process of the first sensor 206a. The signal reflected from the skin and received by the first sensor 206a is used as a feedback for controlling the sensor-skin distance. During operation, the intensity of the sensed signal varies with the distance between the sensor 206a and the skin surface, in which the stronger the sensed signal is, the closer the sensor 206a is to the skin, while the weaker the sensed signal is, the farther the sensor 206a is from the skin. In order to eliminate the effect on the measurement accuracy of the sensed signal caused by the varied distance between the sensor 206a and the skin surface, a constant distance between the sensor 206a and the skin is controlled. Moreover, when the sensor 206a is close to the artery, for example, 1mm-2mm away from the skin surface, the arterial pulsation information could be detected from the sensed signal. By scanning the skin surface along a predetermined path while keeping a constant distance between the sensor 206a and the skin surface within 1mm-2mm, a measuring position range that roughly indicates an artery position is identified according to the analysis of the sensed signal. Once the measuring position range is determined, a position fine-tuning procedure may be performed to determine an accurate location of the artery within the position range for the blood pressure measurement. In one example embodiment, the fine-tuning procedure is carried out by driving the first driving unit 212 and the second driving unit 213. During the position fine-tuning procedure, the second sensor 206b collects a plurality of arterial pulsations under a certain hold-down force from multiple positions within the measuring position range to determine a more accurate measuring position.

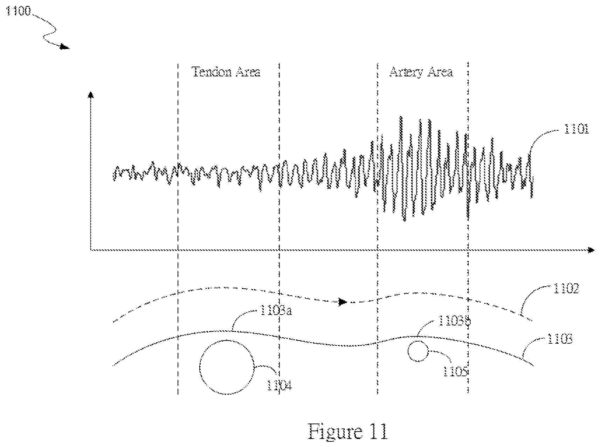

In another example embodiment, as illustrated in Figure 11, 1100, during the non-contact scanning process, the sensed signal 1101 and a scanning trace 1102 of the first sensor 206a will be collected and stored. The scanning trace 1102 of the sensor 206a is used as a representation of a skin contour 1103, as the distance between the sensor 206a and the skin surface is controlled as constant. Once the non-contact scanning process is finished, features of the sensed signal 1101 and the scanning trace 1102 of the sensor 206a can be extracted as inputs for a pre-trained model. In one example embodiment, the pre-trained model is trained and built via machine learning process. The pre-trained model will analyze the sensed signal 1101 and the scanning trace 1102 that represent the skin contour 1103 to predict the artery position for measuring the blood pressure. Usually, the radial artery is under a convex surface of the wrist. However, it may be confused with some protruded front end of tendons. For example, both the skin surfaces 1103a and 1103b above a tendon 1104 and an artery 1105 slightly protrude from the surface. However, the signal with arterial pulsation information sensed within the tendon area is significantly smaller than that within the artery area, as shown in Figure 11. By training with data including the sensed signal, the scanning trace and the corresponding artery position from a number of people, the pre-trained model is developed and able to precisely predict the artery position, where the wrist surface is protruded and the sensed signal with arterial pulsation information is relatively great, based on the variation of the sensed signal 1101 and the scanning trace 1102. In one example embodiment, the accuracy of the measuring position determined via the pre-trained model can be increased to within 1 mm from the artery. Therefore, in another embodiment, the position fine-tuning procedure could be omitted and the blood pressure measurement can be directly conducted within the predicted measuring position.

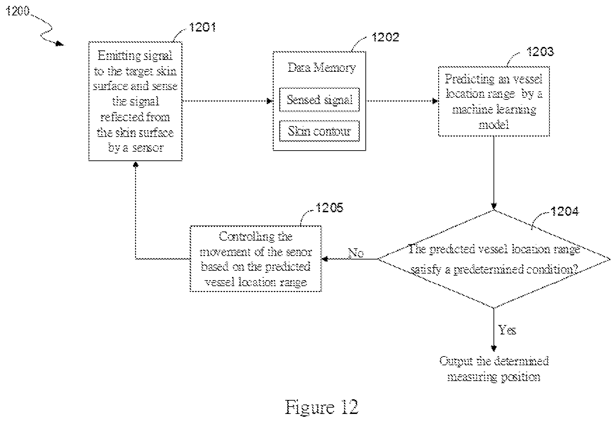

In yet another example embodiment, as illustrated in Figure 12, during the non-contact scanning process, the sensed signal and the scanning trace of the sensor 206a are continuously collected, stored and processed. After the movement of the sensor 206a to a current scanning position as well as the following measurement in step 1201, attributes of the sensed signal and the scanning position are extracted and stored into a data memory for further process in step 1202. In one embodiment, after the movement of the sensor 206a to a current scanning position as well as the following measurement in step 1201, the skin contour is determined based on a series of scanning positions. Thereafter, in step 1203, the sensed data that include the data measured during the current and the prior movements, and the scanning trace that is represented by the current and prior scanning positions of the sensor 206a are sent into a pre-trained model. The pre-trained model will then predict an artery position region based on the current and historical sensed data and the sensing trace in step 1203. If the predicted artery position region satisfies a predetermined condition in step 1204, the predicted artery position region will be output as the identified measuring position for further process. Otherwise, the movement of the sensor 206a to a next scanning position will be controlled based on the predicted artery position range in step 1205, and thereafter, the non-contact scanning process will return back to the step 1201. By adopting this scanning method, it may not be needed to scan the whole predetermined range of wrist surface for identifying the artery position as the scanning process will be completed immediately once the artery position is identified by the pre-trained model. Furthermore, by controlling the movement of the sensor 206a based on the predicted artery position range in real-time, it is not needed to move the sensor 206a step by step along the scanning path, but the sensor 206a can be moved in a varied speed to approach the target artery position more quickly. In one example embodiment, speed and efficiency of identifying the artery location will be significantly increased by using this scanning method.

In one example embodiment, the measuring position is predicted via a machine learning process based on the scanned data and the scan path.

In one embodiment, the pre-trained model will predict the artery position and its confidence range, according to which the next movement of the sensor 206a will be controlled. In one example embodiment, the rate of the movement of the sensor 206a depends on a distance between a current position of the sensor 206a and a possible artery range. For example, when the distance between the current position and the possible artery range is greater than a predetermined threshold (i.e. the sensor 206a is far away from the possible artery range), the sensor will move relatively fast as compared to a case where the distance between the current position and the possible artery range is smaller than a predetermined threshold. Therefore, the efficiency of the non-contact scanning process can be increased. The non-contact scanning process will be terminated as long as the confidence range of the predicted artery position meets an accuracy requirement of the measuring position. In one embodiment, the accuracy requirement is that the confidence range of predict position of artery is smaller than 1-2 mm.

In one example embodiment, the pre-trained model predicting artery position is trained and built based on a large amount of prior non-contact scanning data and correspondingly known artery positions. Attributes extracted from the non-contact scanning data are used as model input X and the known artery position is used as model output Y. The model input X and the model output Y are divided into three sets: a training set that includes X_training and Y_training; a validation set that includes X_validation and Y_validation; and a test set that includes X_test and Y_test. The training and validation sets are used for building model and the test set is used for model performance test. The algorithm of pre-trained model can be, but not limited to, support vector machine, linear regression, or artificial neural network.

Referring back to Figure 3, according to one example embodiment, when the measuring position for the blood pressure measurement on the wrist is identified, the second driving unit 213 (not shown in Figure 3) will control the sensor assembly 206 to move towards and press the wrist surface 205 at the measuring position for measuring the blood pressure of the user. In one example embodiment, the second driving unit 213 includes a controller 308 for controlling the rotation of a gear (not shown in Figure 3) or a gear series 310a and 310b to rotate towards or away from the wrist surface 205, so as to enable the sensor assembly 206, which couples to the gear or gear series 310a and 310b, to move towards and press the wrist surface 205, as shown in Figure 3. In one embodiment, the gear or gear series 310a and 310b are coupled between two guide walls 311 and 312 to prevent the sensor assembly 206 from tilting while pressing the sensor assembly 206 to the wrist surface. In one example embodiment, the guide wall 312 is combined with the controller 308. One of ordinary skill in the art will appreciate that these embodiments are merely examples. For example, the second driving unit 213 could be a mechanical motor such as a pneumatic motor, or an electrical motor such as a step motor or a DC motor, in any configuration with the sensor assembly 206 while satisfying the requirement of driving the sensor assembly 206 to move towards and press the wrist surface for sensing the blood pressure against the wall of the blood vessel. Additionally, the second driving unit 213 could directly or indirectly couples with the sensor assembly 206 for driving the sensor assembly 206 to move towards the wrist surface 205.

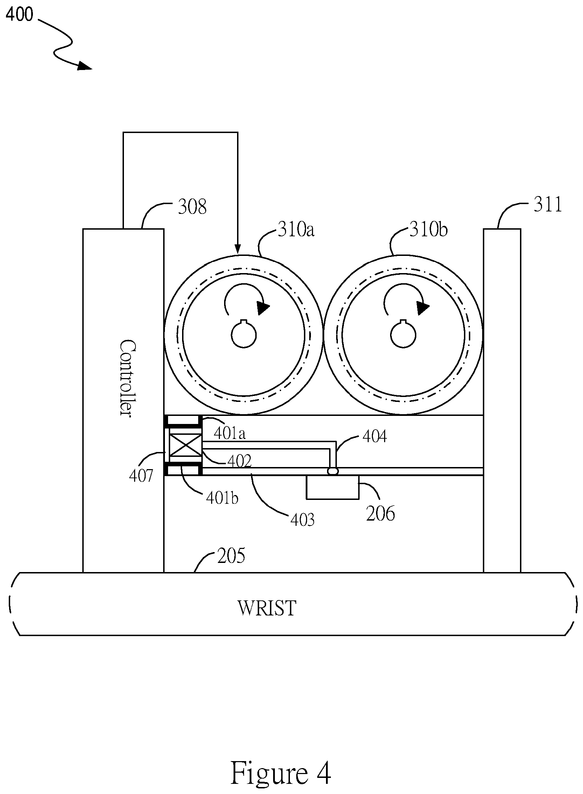

Figure 4 illustrates another schematic drawing of an electronic device 400 for detecting the blood pressure at the internal side of a user's wrist, according to an example embodiment. Figure 4 is described in combination with Figures 2 and 3. Elements with the same or similar reference numerals have the same or similar structure/function as thereof in previous figures. In the electronic device 400 of Figure 4, an electromagnetic motor includes a coil 402 positioned between two magnets 401a and 401b. In one example embodiment, the magnets 401a and 401b are fixed and the coil 402 is movable and connects with a moving unit 404 for driving the moving unit 404 to move along a guiding rail 403 by enabling and adjusting a current flowing through the coil 402. In one embodiment, the moving element 404 is a rolling or sliding element. The moving unit 404 couples with the sensor assembly 206 to bring the sensor assembly 206 to scan the wrist surface 205 in a predetermined path. When the current flows through the coil 402, an electromagnetic force will be generated between the magnets 401a/401b and the coil 402 to enable the coil 402 to move along a direction parallel or substantially parallel to the wrist surface 205. Accordingly, the moving unit 404, which connects to the coil 402, will be driven to move along the guiding rail 403 so as to bring the sensor assembly 206 to scan the wrist surface 205. In an alternative example embodiment, the magnets 401a and 401b are movable and coupled to the moving unit 404 while the coil 402 is fixed. Furthermore, an elastic unit 407 couples with the coil 402 and provides a restoring force to the coil 402. It is understood by one skilled in the art that in addition to the electromagnetic motor as illustrated in Figures 3 and 4, the electromagnetic motor could have other alternative configuration to drive the sensor assembly 206 to scan the wrist surface 205. In one example embodiment, the electromagnetic motor is a VCM motor.

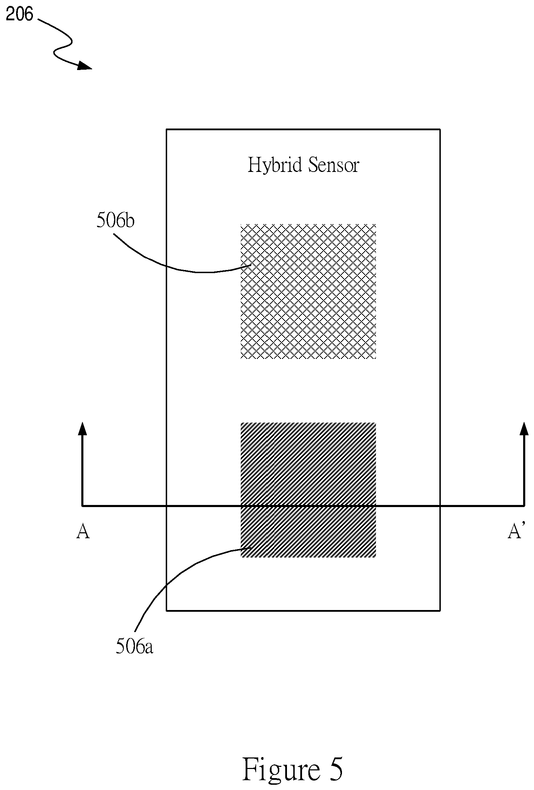

Figure 5 shows a top view of the sensor assembly 206 used in the electronic device 200 (Figure 2), in accordance with an example embodiment. Figure 5 is described in combination with Figure 2. As shown in Figure 5, the sensor assembly 206 is a hybrid sensor assembly that includes two sensors 506a and 506b. The first sensor 506a searches a measuring position by scanning the wrist surface 205 without contacting it. The second sensor 506b measures a blood pressure against a blood vessel wall when blood flows through the blood vessel at the measuring position. In one example embodiment, the blood vessel is an artery. The first sensor 506a and the second sensor 506b dispose on the same side of the sensor assembly 206 that faces the wrist surface 205. A distance between the two sensors 506a and 506b is designed within a predetermined threshold range such that a measurement deviation caused by an offset of the two sensors 506a and 506b is acceptable while the two sensors 506a and 506b are isolated from each other. Furthermore, the first driving unit 212 will adjust a position of the sensor assembly 206 on the wrist surface 205 when the measuring position is identified by the first sensor 506a, so as to locate the second sensor 506b at the measuring position for further process.

During the operation, firstly, the sensor assembly 206 is above the wrist surface 205 and driven to scan the wrist surface 205 along a scanning path to determine a position of a target blood vessel by the first sensor 506a. In one example embodiment, the target blood vessel is a radial artery. When the position of the target blood vessel is identified, the sensor assembly 206 stops moving and stays above the position of the target blood vessel. Then, the sensor assembly 206 is driven to move towards the wrist surface 205 and further press against the wrist surface 205 at the position of the target blood vessel so as to measure the blood pressure by the second sensor 506b.

In one example embodiment, absolute pressure readings can be measured by the second sensor 506b, which is calibrated by a reference force gauge. The blood pressure can be derived or estimated from the measured absolute pressure readings.

In another example embodiment, arterial wall activities can be sensed by the second sensor 506b to generate an arterial pressure pulse waveform, which includes information or attributes of a blood pressure propagation velocity/time along an arterial wall, an arterial pulse reflection velocity/time, and a reflection augmentation index of an arterial pulse, etc. The blood pressure can be derived or estimated from the aforesaid information or attributes extracted from the arterial pressure pulse waveform.

In another example embodiment, blood flow activities can be sensed by the first sensor 506a to generate a blood volume pulse waveform, which includes information or attributes of a blood flow velocity, a blood flow reflection velocity/time, and a reflection augmentation index of the blood flow, etc. In one embodiment, the first sensor 506a emits light toward the wrist surface 205 above the artery and detects the light reflected from the wrist, so as to sense the blood flow activities based on the reflected light that carries the blood information within the blood vessel. The blood pressure can be derived or estimated from the aforesaid information or attributes extracted from the blood volume pulse waveform.

Furthermore, according to an example embodiment, the absolute pressure readings, the information or attributes extracted from the arterial pressure pulse waveform, and/or the information or attributes extracted from the blood volume pulse waveform can be used together to derive or estimate the blood pressure. During the measurements of the absolute pressure readings, the arterial pressure pulse waveform and the blood volume pulse waveform, a hold-down force applied to the sensor assembly 206 for pressing against the skin surface is controlled based on the measured pulse waveforms of the first and second sensors 506a and 506b. In one embodiment, the first sensor 506a is an optical sensor and the second sensor 506b is a pressure sensor.

Moreover, as there are much less blood capillaries under the skin surface of the wrist, it is more difficult to measure blood oxygen saturation via the blood capillaries at the wrist as compared to measuring at a finger. Under such conditions, to measure the blood oxygen saturation via the radial artery is a solution as the radial artery is near the wrist surface with increased blood flow. Unfortunately, at the skin surface above radial artery, the mechanical pulsation is so strong that it will affect the reflected pulsations of red and infra-red light and affect the measurement accuracy of pulse oximetry. In one example embodiment, the sensor assembly 206 integrated with the optical sensor and the pressure sensor can be used to accurately measure the blood oxygen saturation at the radial artery.

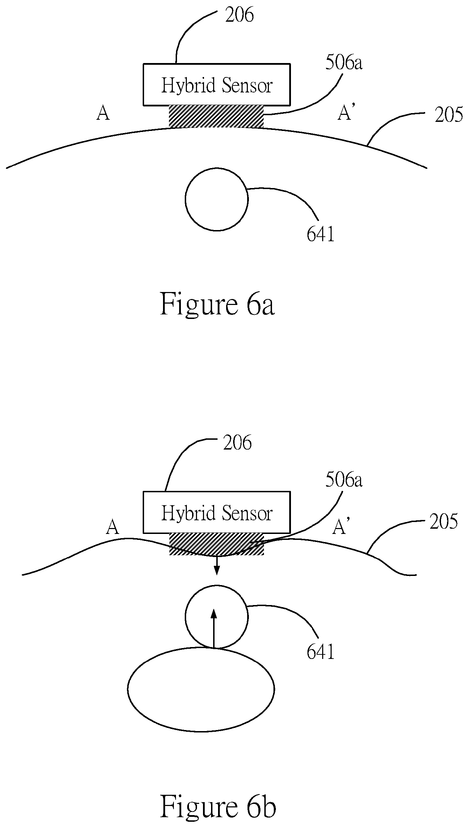

Figures 6A and 6B show an operating mechanism of the electronic device 200 with the sensor assembly 206, according to one example embodiment. Figures 6A and 6B are described in combination with Figures 2 and 5. The cross-sectional view of the sensor assembly 206 in Figures 6A and 6B is derived from the line A-A' of Figure 5. The sensor assembly 206 is controlled by the first driving unit 212 and second driving unit 213 as described in Figure 2.

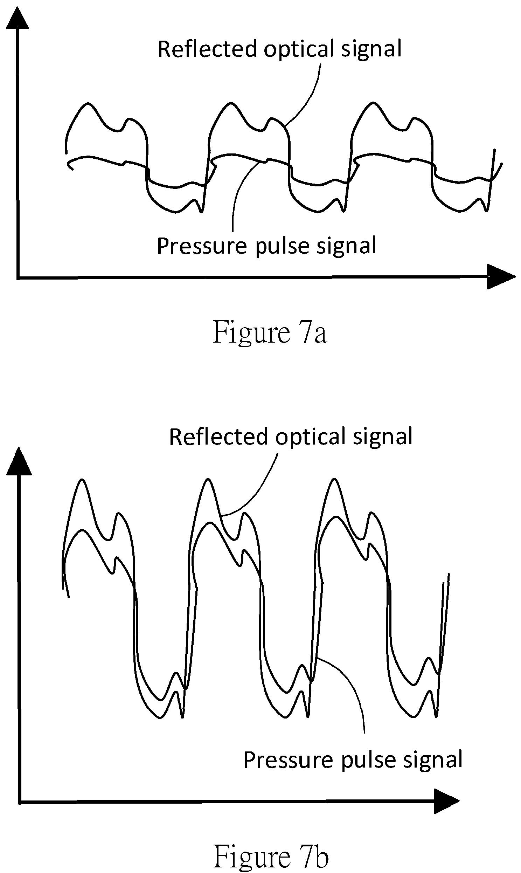

During operation, when the optical sensor 506a identifies the measuring position of the radial artery 641 at the wrist, the sensor assembly 206 will be moved towards the wrist surface 205 at the identified measuring location. Referring to Figure 6A, when the sensor assembly 206 is driven to move towards the wrist surface 205 at the identified measuring location and touches on the wrist surface 205, an optical signal reflected from the wrist surface 205, so called photoplethysmorgraphy (PPG), as shown in Figure 7A that carries information of blood volume changes can be detected by the optical sensor 506a for the calculation of blood oxygen saturation. In one embodiment, the optical sensor 506a emits light towards the wrist and detects the PPG signal from the wrist. Referring to Figure 6B, after touching on the wrist surface 205, the sensor assembly 206 will continue to move towards and press against the wrist surface 205 over the location of the radial artery 641 by a predetermined hold-down force until the sensor assembly 206 reaches a predetermined depth for blood pressure measurement.

Figure 7A shows waveforms of a reflected optical signal and pressure pulse signal detected by the sensor assembly 206 when it is in touch with the wrist surface 205 as illustrated in Figure 6A, according to one example embodiment. Figure 7B shows waveforms of the reflected optical signal and pressure pulse signal detected by the sensor assembly 206 when it presses against the wrist surface 205 as illustrated in Figure 6B, according to one example embodiment. During the operation, the blood oxygen saturation of the user is calculated based on the optical signal reflected from the wrist surface 205 and detected by the sensor assembly 206. More specifically, the blood oxygen saturation is calculated based on a ratio of the AC part to DC part of the optical signal. The AC part of the optical signal is a variable part containing changes caused by both mechanical variation and blood flow. In order to obtain an accurate measurement result of the blood oxygen saturation, it is important to eliminate the effect of the mechanical variation applied to the AC part of the optical signal.

By comparing Figure 7B with Figure 7A, although the AC part of optical signal in Figure 7B is stronger than in Figure 7A, the increase of AC intensity is mainly induced by mechanical pulsation, as the skin tissue resonance with arterial pulsation is gradually increased when the sensor assembly 206 is pressed towards the radial artery 641, according to an example embodiment. Hence, the measurement accuracy of blood oxygen saturation is affected accordingly. In order to eliminate the influence of mechanical pulsation of the radial artery, it is preferred to avoid deeply pressing the sensor assembly 206 against the radial artery. In another aspect, since the light leakage caused by the gap between the sensor assembly 206 and the wrist surface may also affect the measurement accuracy, the sensor assembly 206 is close to the skin surface to avoid light leakage during the measurement of the blood oxygen saturation. Therefore, on controlling the pressing of the sensor assembly 206 against the wrist surface 205, an optimal contact depth of the sensor assembly 206 upon the wrist surface is determined to balance the impact on the measurement accuracy of the blood oxygen saturation caused by the mechanical pulsation of the radial artery and the light leakage.

Furthermore, as shown in Figures 7A and 7B, according to one example embodiment, the pressure pulse signal increases with the increment of a pressed depth of the sensor assembly 206 against the wrist surface 205. In other words, the pressure pulse signal varies with the pressed depth of the sensor assembly 206 against the wrist surface 205. Therefore, the contact depth of the sensor assembly 206 upon the wrist surface could be controlled based on the detected pressure pulse signal to maintain the sensor assembly 206 at the optimal contact depth.

In one example embodiment, when the sensor assembly 206 presses against the wrist surface 205, the pressure pulse between the sensor assembly 206 and the wrist is monitored by the pressure sensor 506b (Figure 5) to control the hold-down force applied on the sensor assembly 206. To minimize the impact on the measurement accuracy of the blood oxygen saturation caused by the mechanical pulsation and avoid light leakage, the optical sensor 506a will measure the blood oxygen saturation when the pressure pulse is between 0 - 40 mmHg. In other words, the optimal situation to measure the blood oxygen saturation is when the sensor assembly 206 just touches or slightly press the wrist surface 205. By monitoring the pressure pulse sensed by the pressure sensor 506b, the optimal situation could be identified and maintained by adjusting the hold-down force applied on the sensor assembly 206.

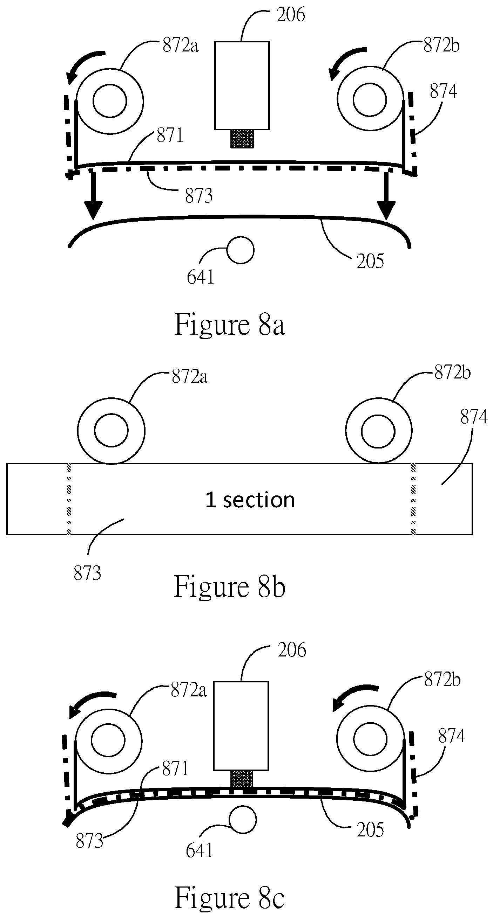

In one example embodiment, to avoid the sensor assembly 206 from contacting the skin surface directly, a membrane is covered on the measuring surface of the sensor assembly 206 to isolate the sensor assembly 206 from the skin surface. Figure 8A illustrates an exemplary schematic structure of the electronic device 200 with a membrane unit, in accordance with one example embodiment of the presented invention. Referring to Figure 8A, a section of membrane is added to cover a measuring surface 871 of the electronic device in order to isolate the measuring surface 871 from the user's skin surface 205. To facilitate the user, at least one rolling element that rolls multiple membrane sections one by one is disposed inside the electronic device. In one example embodiment as illustrated in Figure 8A, the electronic device includes two rolling elements 872a and 872b. In a further example embodiment, the rolling elements 872a and 872b for rolling the membrane sections are controlled manually by the user or automatically by an individual motor controller. In another example embodiment, the rolling elements 872a and 872b are integrated with the first driving unit 212 or the second driving unit 213 (Figure 2) for rolling the membrane sections. In one example embodiment, the rolling elements 872a and 872b are controlled by the first driving unit 212 and/or the second driving unit 213 for rolling the membrane sections.

In each new measurement, a new membrane section 873 of the membrane unit will be rolled out to cover the measuring surface 871 and a used section 874 will be rolled into the device for withdrawn as specified in Figure 8A. In one embodiment, the new membrane sections 873 of the membrane unit are stored at one side of the electronic device 200 and the used membrane sections 874 are withdrawn and stored at another side of the electronic device 200.

Figure 8B shows the new membrane section 873 of the membrane unit from a bottom view, in accordance with one example embodiment. During operation, before each new measurement is made, the used membrane section 874 is rolled back into the electronic device and the new membrane section 873 is consequently rolled out to cover the measuring surface 871. When the electronic device 200 is worn on the user's wrist, the new membrane section 873 will be adhered to the skin surface 205 to prevent the measuring surface 871 of the electronic device 200 from directly contacting the skins of different users while improving the stability of the electronic device 200 as shown in Figure 8C, so as to avoid cross-contamination between different users.

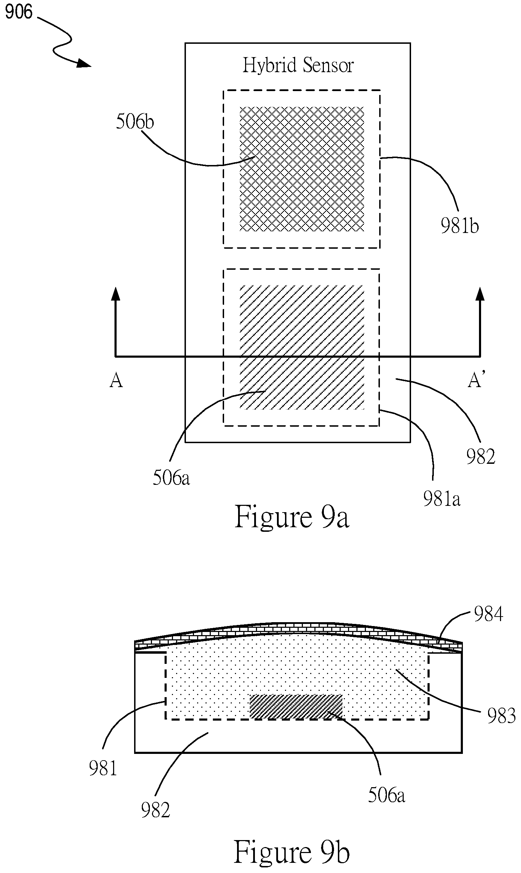

Additionally, Figure 9A shows a top view of a sensor assembly 906 with a coating layer, according to one example embodiment. Figure 9B shows a cross-sectional view (from an A-A' direction of Figure 9A) of the sensor assembly 906 with the coating layer, in accordance with an example embodiment. Figures 9A and 9B are described in combination with Figure 5. As shown in Figure 9A, the first sensor 506a and the second sensor 506b are respectively disposed in a first sensor cavity 981a and a second sensor cavity 981b, which are embedded in a substrate 982 of the sensor assembly 906. More specifically, as referring to Figures 9A and 9B, the first sensor 506a disposes at a bottom of the first sensor cavity 981a. A transparent material is filled in the first sensor cavity 981a to encapsulate the first sensor 506a so as to protect and prevent the first sensor 506a from directly contacting the outside. In one example embodiment, the transparent material forms an encapsulate layer 983 for the first sensor 506a. Furthermore, a protecting layer 984 is coated on a surface of the sensor assembly 906 in order to not only reduce the friction between the wrist surface 205 and the sensor assembly 906, but also minimize diffusion of moisture into the encapsulate layers of the respective sensors, for example, the encapsulate layer 983 of the first sensor 506a, so as to enhance the reliability of the whole sensor assembly 906. In one example embodiment, the protecting layer 984 is sprayed on to the whole surface of the sensor assembly 906. Configuration of the second sensor 506b within the second sensor cavity 981b could have similar structure as that of the first sensor 506a within the first sensor cavity 981a, as illustrated by Figure 9b.

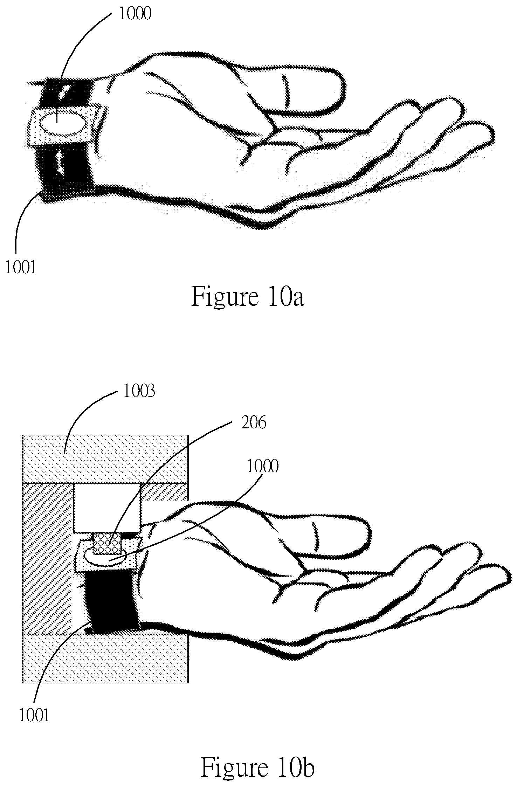

Figure 10A shows a movable frame 1000 being worn on a user's wrist via a wristband, according to one example embodiment. Figure 10B shows a portable device that couples to the movable frame 1000 for measuring the health information of the user, according to an alternative example embodiment of the present invention. In the example embodiment shown in Figure 10A, in order to minimize the weight of the device frequently worn on the wrist, a movable frame 1000 is worn on the wrist of a user for receiving the sensor assembly 206, in which the sensor assembly could be attached to and detached from the movable frame 1000. In one example embodiment, the movable frame 1000 is worn on the wrist via a wristband 1001, as shown in Figure 10A. In another example embodiment, the movable frame 1000 could be worn on the wrist via gloves, mittens or in other wearable styles. For measuring the health information of the user, as shown in Figure 10B, a portable device 1003 that includes the sensor assembly 206, the first driving unit 212 and the second driving unit 213 (not shown on Figure 10B) is disposed on the wrist and the sensor assembly 206 is coupled to the movable frame 1000 manually or automatically. In one example embodiment, the first driving unit 212, the second driving unit 213 and the sensor assembly 206 are detachable from the portable device 1003. In another embodiment, the second driving unit 213 integrates with the sensor assembly 206 and the first driving unit 212 is detachable from the portable device 1003. In yet another embodiment, the first driving unit 212, the second driving unit 213 and the sensor assembly 206 are integrated together with the portable device 1003.

During operation, the first driving unit 212 drives the sensor assembly 206, which couples with the frame 1000, to scan the wrist's skin surface to determine a suitable position for measurement. Then, the first driving unit 212 is detached from the portable device 1003 for load release and the second driving unit 213 will drive the sensor assembly 206 along with the frame 1000 to move towards the wrist skin at the suitable position in order to perform pulse oximetry and blood pressure measurements, in one example embodiment. In another example embodiment, when the suitable position is identified, the second driving unit 213 will start to control the movement of the sensor assembly 206 with the frame 1000 towards the wrist surface without detaching the first driving unit 212 from the portable device 1003. Additionally, when the suitable position is identified, the movable frame 1000 could be locked at the identified position to prevent the displacement/offset of the sensor assembly 206 along the wrist surface during measurement.

After measurement, the portable device 1003 is detached from the wrist to release the load on the user's wrist. In another example embodiment, the sensor assembly 206 is always fixed with the movable frame 1000 to be worn on the user's wrist. For measuring the health information, the portable device 1003 with the two driving units 212 and 213 is coupled to the sensor assembly 206 to control the movement of the sensor assembly 206 so as to achieve the measurement as described above.

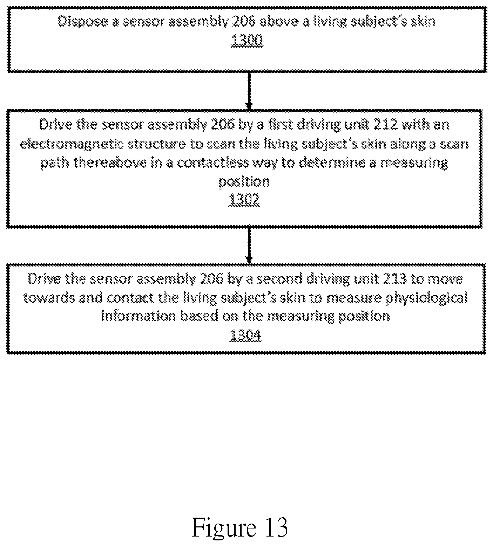

In one example embodiment, Figure 13 shows a flowchart of an electronic device being applied to a living subject for healthcare measurement. Figure 13 is described in combination with Figure 2. A sensor assembly 206 is disposed above a living subject's skin in box 1300. The sensor assembly 206 is driven by a first driving unit 212 with an electromagnetic structure to scan the living subject's skin along a moving element there above in a contactless way to determine a measuring position in box 1302. The sensor assembly 206 is driven by a second driving unit 213 to move towards and contact the living subject's skin to measure physiological information based on the measuring position in box 1304.

In one example embodiment, a magnet interacts with a coil of the first driving unit to generate an electromagnetic force for driving the sensor assembly.

In another example embodiment, a sliding element is moved by a moving element along a guiding rail. In yet another embodiment, a friction force is generated between the guiding rail and the moving element during the movement to reduce the shift and improve the stability of the sensor assembly.

## Claims

1. An electronic device (200,300) configured to measure physiological information of a living subject, the electronic device (200,300) comprising:
a sensor assembly (206),
a first driving unit (212) configured to drive the sensor assembly (206) to scan the living subject's skin along a scan path there-above in a contactless way to determine a measuring position; and
a second driving unit (213) configured to drive the sensor assembly (206) to move towards and contact the living subject's skin to measure the physiological information based on the measuring position;
**characterised in that** it further comprises a supporting unit (214) configured so that the electronic device (200,300) is mountable on the inner side (205) of a wrist (216) via the supporting unit (214);
and **in that** the first driving unit (212) includes at least one magnet (301a) and at least one coil (302a) configured to interact with the at least one magnet (301a) for generating a magnetic force to enable the at least one coil (302a) to move relative to the at least one magnet (301a) along a guiding rail.

2. The electronic device (200,300) of claim 1, configured so that a distance between the sensor assembly (206) and the living subject's skin is controllable to be constant.

3. The electronic device (200,300) of claim 1, wherein the magnet (301a) and the coil (302a) interact to generate an electromagnetic force through interaction to drive the sensor assembly (206); or the first driving unit (212) comprises a moving element (304) that couples to the sensor assembly (206) and a guiding rail (303) for guiding the moving element (304) to move along the guiding rail (303).

4. The electronic device (200,300) of claim 1, wherein the second driving unit (213) comprises:
a controller; and
at least one gear that couples to the sensor assembly (206);
wherein the controller is configured to control rotation of the gear to rotate towards or away from the living subject's skin, so as to enable the sensor assembly (206) that couples to the gear to move towards the living subject's skin for the measurement.

5. The electronic device (200,300) of claim 1, wherein the second driving unit (213) comprises:
two guide walls (311,312); and
two gears (310a,310b),
wherein the two gears are coupled side by side between the two guide walls (311,312) to press the sensor assembly (206) towards the living subject's skin and prevent the sensor assembly (206) from tilting.

6. The electronic device (200,300) of claim 1, wherein the sensor assembly (206) is operable to fine tune the measuring position by measuring pressure pulse signals at multiple positions nearby the measuring position and determine an optimal position based on the measured pressure pulse signals.

7. The electronic device (200,300) of claim 1, wherein the sensor assembly (206) is operable to detect a blood oxygen saturation of the living subject at the measuring position, and a contact depth of the sensor assembly (206) upon the living subject's skin during the detection of the blood oxygen saturation is controlled based on the measured physiological information.

8. The electronic device (200,300) of claim 1, wherein the measuring position is predicted via a prediction algorithm based on the scan path and scanned signals sensed along the scan path.

9. The electronic device (200,300) of claim 1, wherein the first driving unit (212) is configured to drive the sensor assembly (206) to scan the living subject's skin in the contactless way to determine the measuring position by:
generating a predicted measuring position based on the sensed signals measured at a current and prior scanning positions of the sensor assembly (206) by a pre-trained model; and
outputting the predicted measuring position for further process if the predicted measuring position satisfies a predetermined condition; and
controlling movement of the sensor assembly (206) to a next scanning position based on the predicted measuring position if the predicted measuring position does not satisfy the predetermined condition, and returning to the emitting step.

10. A method that applies an electronic device (200,300) according to any of claims 1-9 to a living subject, the method comprising:
disposing a sensor assembly (206) above the living subject's skin;
mounting the electronic device (200,300), by a supporting unit (214), on the inner side (205) of a wrist (216);
driving the sensor assembly (206), by a first driving unit (212), to scan the living subject's skin along a scan path there-above in a contactless way to determine a measuring position, wherein the first driving unit (212) includes at least one magnet (301a) and at least one coil (302a) that interacts with the at least one magnet (301a) for generating a magnetic force to enable the at least one coil (302a) to move relative to the at least one magnet (301a) along a guiding rail; and
driving the sensor assembly (206), by a second driving unit (213), to move towards and contact the living subject's skin to measure physiological information based on the measuring position.

11. The method of claim 10, wherein a distance between the sensor assembly and the living subject's skin is controllable to be constant.

12. The method of claim 10, further comprising:
fine-tuning the measuring position by measuring pressure pulse signals at multiple positions nearby the measuring position; and
determining an optimal position based on the measured pressure pulse signals.

13. The method of claim 10, further comprising:
detecting a blood oxygen saturation of the living subject at the measuring position by the sensor assembly (206), and
controlling a contact depth of the sensor assembly (206) upon the living subject's skin during the detection of the blood oxygen saturation based on the measured physiological information.

14. The method of claim 10, further comprising predicting the measuring position by a prediction algorithm based on the scanned path and scanned signals sensed along the scan path.

15. The method of claim 10, wherein the measuring position is determined by:
generating a predicted measuring position based on the sensed signals measured at a current and prior scanning positions of the sensor assembly (206) by a pre-trained model;
outputting the predicted measuring position for further process if the predicted measuring position satisfies a predetermined condition; and
controlling movement of the sensor assembly (206) to a next scanning position based on the predicted measuring position if the predicted measuring position does not satisfy a predetermined condition, and returning to the emitting step.

## Patentansprüche

1. Ein elektronisches Gerät (200, 300), das konfiguriert ist, um physiologische Informationen eines lebenden Subjekts zu messen, wobei das elektronische Gerät (200, 300) Folgendes umfasst:
eine Sensoranordnung (206),
eine erste Antriebseinheit (212), die so konfiguriert ist, dass sie die Sensoranordnung (206) antreibt, um die Haut des lebenden Subjekts entlang eines Abtastpfades darüber berührungslos abzutasten, um eine Messposition zu bestimmen; und
eine zweite Antriebseinheit (213), die so konfiguriert ist, dass sie die Sensoranordnung (206) so antreibt, dass sie sich auf die Haut des lebenden Subjekts zubewegt und diese berührt, um die physiologischen Informationen auf der Grundlage der Messposition zu messen;
**dadurch gekennzeichnet, dass** es ferner eine Stützeinheit (214) umfasst, die so konfiguriert ist, dass das elektronische Gerät (200, 300) über die Stützeinheit (214) an der Innenseite (205) eines Handgelenks (216) angebracht werden kann;
und dass die erste Antriebseinheit (212) mindestens einen Magneten (301a) und mindestens eine Spule (302a) umfasst,
die so konfiguriert ist, dass sie mit dem mindestens einen Magneten (301a) zusammenwirkt, um eine Magnetkraft zu erzeugen, damit sich die mindestens eine Spule (302a) relativ zu dem mindestens einen Magneten (301a) entlang einer Führungsschiene bewegen kann.

2. Das elektronische Gerät (200, 300) nach Anspruch 1, das so konfiguriert ist, dass ein konstanter Abstand zwischen der Sensoranordnung (206) und der Haut des lebenden Subjekts steuerbar ist.

3. Das elektronische Gerät (200, 300) nach Anspruch 1, wobei der Magnet (301a) und die Spule (302a) zusammenwirken, um durch Interaktion eine elektromagnetische Kraft zu erzeugen, um die Sensoranordnung (206) anzutreiben; oder wobei die erste Antriebseinheit (212) ein bewegliches Element (304) ist, das mit der Sensoranordnung (206) gekoppelt ist, und eine Führungsschiene (303) umfasst, um das bewegliche Element (304) so zu führen, dass es sich entlang der Führungsschiene (303) bewegt.

4. Das elektronische Gerät (200, 300) nach Anspruch 1, wobei die zweite Antriebseinheit (213) Folgendes umfasst:
eine Steuerung; und
mindestens ein Getriebe, das mit der Sensoranordnung (206) gekoppelt ist;
wobei die Steuereinheit so konfiguriert ist, dass sie die Drehung des Zahnrads so steuert, dass es sich zur Haut des lebenden Subjekts hin oder von ihr weg dreht, so dass sich die Sensoranordnung (206), die mit dem Zahnrad gekoppelt ist, für die Messung zur Haut des lebenden Subjekts hin bewegen kann.

5. Das elektronische Gerät (200, 300) nach Anspruch 1, wobei die zweite Antriebseinheit (213) Folgendes umfasst:
zwei Führungswände (311, 312); und
zwei Zahnräder (310a, 310b),
wobei die beiden Zahnräder nebeneinander zwischen den beiden Führungswänden (311, 312) gekoppelt sind, um die Sensoranordnung (206) gegen die Haut des lebenden Subjekts zu drücken und ein Kippen der Sensoranordnung (206) zu verhindern.

6. Das elektronische Gerät (200, 300) nach Anspruch 1, wobei die Sensoranordnung (206) zur Feinabstimmung der Messposition durch Messung von Druckimpulssignalen an mehreren Positionen in der Nähe der Messposition und zur Bestimmung einer optimalen Position auf der Grundlage der gemessenen Druckimpulssignale betreibbar ist.

7. Das elektronische Gerät (200, 300) nach Anspruch 1, wobei die Sensoranordnung (206) so betreibbar ist, dass sie eine Blutsauerstoffsättigung des lebenden Subjekts an der Messposition erfasst und eine Kontakttiefe der Sensoranordnung (206) auf der Haut des lebenden Subjekts während der Erfassung der Blutsauerstoffsättigung auf der Grundlage der gemessenen physiologischen Informationen gesteuert wird.

8. Das elektronische Gerät (200, 300) nach Anspruch 1, wobei die Messposition über einen Vorhersagealgorithmus auf der Grundlage des Abtastpfades und der entlang des Abtastpfades erfassten Abtastsignale vorhergesagt wird.

9. Das elektronische Gerät (200, 300) nach Anspruch 1, wobei die erste Antriebseinheit (212) so konfiguriert ist, dass sie die Sensoranordnung (206) antreibt, um die Haut des lebenden Subjekts berührungslos abzutasten, um die Messposition zu bestimmen, indem sie:
eine vorhergesagte Messposition auf der Grundlage der erfassten Signale erzeugt, die an einer aktuellen und früheren Abtastpositionen der Sensoranordnung (206) gemessen wurden, durch ein vorab trainiertes Modell; und
die vorhergesagte Messposition zur weiteren Verarbeitung ausgibt, wenn die vorhergesagte Messposition eine vorbestimmte Bedingung erfüllt; und
die Bewegung der Sensoranordnung (206) zu einer nächsten Abtastposition auf der Grundlage der vorhergesagten Messposition steuert, wenn die vorhergesagte Messposition die vorbestimmte Bedingung nicht erfüllt, und zum Sendeschritt zurückkehrt.

10. Ein Verfahren zur Anwendung eines elektronischen Geräts (200, 300) nach einem der Ansprüche 1-9 an einem lebenden Subjekt, wobei das Verfahren Folgendes umfasst Anordnen einer Sensoranordnung (206) über der Haut des lebenden Subjekts;
Anbringen des elektronischen Geräts (200, 300) mittels einer Stützeinheit (214) an der Innenseite (205) eines Handgelenks (216);
Antreiben der Sensoranordnung (206) durch eine erste Antriebseinheit (212), um die Haut des lebenden Subjekts entlang eines Abtastpfades darüber berührungslos abzutasten, um eine Messposition zu bestimmen, wobei die erste Antriebseinheit (212) mindestens einen Magneten (301a) und mindestens eine Spule (302a) enthält, die mit dem mindestens einen Magneten (301a) zusammenwirkt, um eine Magnetkraft zu erzeugen, damit sich die mindestens eine Spule (302a) relativ zu dem mindestens einen Magneten (301a) entlang einer Führungsschiene bewegen kann; und
Antreiben der Sensoranordnung (206) durch eine zweite Antriebseinheit (213), um sich auf der Haut des lebenden Subjekts zuzubewegen und diese zu berühren, um physiologische Informationen auf der Grundlage der Messposition zu messen.

11. Das Verfahren nach Anspruch 10, wobei ein konstanter Abstand zwischen der Sensoranordnung und der Haut des lebenden Subjekts steuerbar ist.

12. Das Verfahren nach Anspruch 10, welches ferner Folgendes umfasst:
Feinabstimmung der Messposition durch Messen von Druckimpulssignalen an mehreren Positionen in der Nähe der Messposition; und
das Bestimmen einer optimalen Position auf der Grundlage der gemessenen Druckimpulssignale.

13. Das Verfahren nach Anspruch 10, welches ferner Folgendes umfasst:
Erfassen einer Blutsauerstoffsättigung des lebenden Subjekts an der Messposition durch die Sensoranordnung (206), und
Steuern einer Kontakttiefe der Sensoranordnung (206) auf der Haut des lebenden Subjekts während der Erfassung der Blutsauerstoffsättigung auf der Grundlage der gemessenen physiologischen Informationen.

14. Das Verfahren nach Anspruch 10, das ferner die Vorhersage der Messposition durch einen Vorhersagealgorithmus auf der Grundlage des Abtastpfades und der entlang des Abtastpfades erfassten Abtastsignale umfasst.

15. Das Verfahren nach Anspruch 10, wobei die Messposition durch Folgendes bestimmt wird:
Erzeugen einer vorhergesagte Messposition auf der Grundlage der erfassten Signale, die an einer aktuellen und früheren Abtastpositionen der Sensoranordnung (206) gemessen wurden, durch ein vorab trainiertes Modell;
Ausgeben der vorhergesagten Messposition zur weiteren Verarbeitung, wenn die vorhergesagte Messposition eine vorbestimmte Bedingung erfüllt; und
Steuern der Bewegung der Sensoranordnung (206) zu einer nächsten Abtastposition auf der Grundlage der vorhergesagten Messposition, wenn die vorhergesagte Messposition eine vorbestimmte Bedingung nicht erfüllt, und zum Sendeschritt zurückkehrt.

## Revendications

1. Dispositif électronique (200,300) configuré pour mesurer les informations physiologiques d'un sujet vivant, le
dispositif électronique (200,300) comprenant :
un ensemble capteur (206),
une première unité d'entraînement (212) configurée pour entraîner l'ensemble capteur (206) afin de balayer la peau du sujet vivant le long d'une trajectoire de balayage au-dessus de celle-ci, sans contact, afin de déterminer une position de mesure ; et
une seconde unité d'entraînement (213) configurée pour entraîner l'ensemble capteur (206) pour qu'il se déplace vers la peau du sujet vivant et entre en contact avec celle-ci afin de mesurer les informations physiologiques sur la base de la position de mesure ;
**caractérisé en ce qu'**il comprend en outre une unité de support (214) conçue de sorte que le dispositif électronique (200,300) puisse être monté sur la face interne (205) d'un poignet (216) par l'intermédiaire de l'unité de support (214) ;
et **en ce que** la première unité d'entraînement (212) comporte au moins un aimant (301a) et au moins une bobine (302a) configurée pour interagir avec l'au moins un aimant (301a) afin de générer une force magnétique permettant à l'au moins une bobine (302a) de se déplacer par rapport à l'au moins un aimant (301a) le long d'un rail de guidage.

2. Dispositif électronique (200,300) selon la revendication 1, configuré de sorte qu'une distance entre l'ensemble capteur (206) et la peau du sujet vivant peut être contrôlée pour être constante.

3. Dispositif électronique (200,300) selon la revendication 1, dans lequel l'aimant (301a) et la bobine (302a) interagissent pour générer une force électromagnétique par interaction afin d'entraîner l'ensemble capteur (206) ; ou la première unité d'entraînement (212) comprend un élément mobile (304) qui s'accouple à l'ensemble capteur (206) et un rail de guidage (303) pour guider l'élément mobile (304) afin qu'il se déplace le long du rail de guidage (303).

4. Dispositif électronique (200,300) selon la revendication 1, dans lequel la seconde unité d'entraînement (213) comprend :
un dispositif de commande ; et
au moins un engrenage qui s'accouple à l'ensemble capteur (206) ;
dans lequel le dispositif de commande est configuré pour commander la rotation de l'engrenage afin qu'il se déplace par rotation vers ou à l'écart de la peau du sujet vivant, de manière à permettre à l'ensemble capteur (206) qui s'accouple à l'engrenage de se déplacer vers la peau du sujet vivant pour effectuer la mesure.

5. Dispositif électronique (200,300) selon la revendication 1, dans lequel la seconde unité d'entraînement (213) comprend :
deux parois de guidage (311,312) ; et
deux engrenages (310a,310b),
dans lequel les deux engrenages sont accouplés côte à côte entre les deux parois de guidage (311,312) pour presser l'ensemble capteur (206) contre la peau du sujet vivant et empêcher l'ensemble capteur (206) de basculer.

6. Dispositif électronique (200,300) selon la revendication 1, dans lequel l'ensemble capteur (206) permet d'effectuer un réglage fin de la position de mesure en mesurant les signaux d'impulsion de pression à plusieurs positions proches de la position de mesure et de déterminer une position optimale sur la base des signaux d'impulsion de pression mesurés.

7. Dispositif électronique (200,300) selon la revendication 1, dans lequel l'ensemble capteur (206) permet de détecter une saturation en oxygène du sang du sujet vivant à la position de mesure, et une profondeur de contact de l'ensemble capteur (206) sur la peau du sujet vivant pendant la détection de la saturation en oxygène du sang est contrôlée sur la base des informations physiologiques mesurées.

8. Dispositif électronique (200,300) selon la revendication **1,** dans lequel la position de mesure est prédite par le biais d'un algorithme de prédiction sur la base de la trajectoire de balayage et des signaux balayés détectés le long de la trajectoire de balayage.

9. Dispositif électronique (200,300) selon la revendication 1, dans lequel la première unité d'entraînement (212) est configurée pour commander l'ensemble capteur (206) afin de balayer la peau du sujet vivant, sans contact, pour déterminer la position de mesure par :
la génération d'une position de mesure prédite sur la base des signaux détectés mesurés à une position de balayage actuelle et antérieure de l'ensemble capteur (206) par un modèle pré-entraîné ; et
l'émission de la position de mesure prédite en vue d'un traitement ultérieur si la position de mesure prédite satisfait à une condition prédéterminée ; et
la commande du déplacement de l'ensemble capteur (206) vers une position de balayage suivante sur la base de la position de mesure prédite si la position de mesure prédite ne satisfait pas à la condition prédéterminée, et le retour à l'étape d'émission.

10. Procédé d'application d'un dispositif électronique (200,300) selon l'une quelconque des revendications 1-9 à un sujet vivant, le procédé comprenant :
le placement d'un ensemble capteur (206) au-dessus de la peau du sujet vivant ;
le montage du dispositif électronique (200,300), par une unité de support (214), sur la face interne (205) d'un poignet (216) ;
l'entraînement de l'ensemble capteur (206), par une première unité d'entraînement (212), pour balayer la peau du sujet vivant le long d'une trajectoire de balayage au-dessus celle-ci, sans contact, afin de déterminer une position de mesure, dans lequel la première unité d'entraînement (212) comporte au moins un aimant (301a) et au moins une bobine (302a) qui interagit avec l'au moins un aimant (301a) pour générer une force magnétique afin de permettre à l'au moins une bobine (302a) de se déplacer par rapport à l'au moins un aimant (301a) le long d'un rail de guidage ; et
l'entraînement de l'ensemble capteur (206), par une seconde unité d'entraînement (213), pour qu'il se déplace vers la peau du sujet vivant et entre en contact avec celle-ci afin de mesurer des informations physiologiques sur la base de la position de mesure.

11. Procédé selon la revendication 10, dans lequel une distance entre l'ensemble capteur et la peau du sujet vivant peut être contrôlée pour être constante.

12. Procédé selon la revendication 10, comprenant en outre :
le réglage fin de la position de mesure en mesurant les signaux d'impulsion de pression à plusieurs positions proches de la position de mesure ; et
la détermination d'une position optimale sur la base des signaux d'impulsion de pression mesurés.

13. Procédé selon la revendication 10, comprenant en outre :
la détection de la saturation en oxygène du sang du sujet vivant à la position de mesure par l'ensemble capteur (206), et
le contrôle d'une profondeur de contact de l'ensemble capteur (206) sur la peau du sujet vivant pendant la détection de la saturation en oxygène du sang sur la base des informations physiologiques mesurées.

14. Procédé selon la revendication 10, comprenant en outre la prédiction de la position de mesure par un algorithme de prédiction sur la base de la trajectoire de balayage et des signaux de balayage détectés le long de la trajectoire de balayage.

15. Procédé selon la revendication 10, dans lequel la position de mesure est déterminée par :
la génération d'une position de mesure prédite sur la base des signaux détectés mesurés à une position de balayage actuelle et antérieure de l'ensemble capteur (206) par un modèle pré-entraîné ;
l'émission de la position de mesure prédite en vue d'un traitement ultérieur si la position de mesure prédite satisfait à une condition prédéterminée ; et
la commande du déplacement de l'ensemble capteur (206) vers une position de balayage suivante sur la base de la position de mesure prédite si la position de mesure prédite ne satisfait pas à une condition prédéterminée, et le retour à l'étape d'émission.
